# FASCICULE DE BREVET EUROPEEN

(11) **EP 2 605 757 B1**
(45) Date de publication et mention de la délivrance du brevet: **01.07.2015**
(21) Numéro de dépôt: 11758519.0
(22) Date de dépôt: 18.08.2011
(51) Int. Cl.: A61K 9/20, A61K 9/28, A61K 9/50, A61K 31/485, A61K 47/10, A61K 47/36, A61K 47/26, A61K 47/02, A61P 25/04

(54) **FORMULATIONS A BASE DE NALBUPHINE ET LEURS UTILISATIONS**
AUF NALBUPHIN BASIERENDE FORMULUERUNGEN UND IHRE VERWENDUNG
NALBUPHINE-BASED FORMULATIONS AND USES THEREOF

(30) Priorité: 20.08.2010 FR 1056689
(43) Date de publication de la demande: 26.06.2013
(73) Titulaire: Debregeas Et Associes Pharma, 75008 Paris (FR)
(72) Inventeur: LEBON, Christophe, F-28260 Rouvres (FR); SUPLIE, Pascal, F-27400 Montaure (FR); PAUL, David Olivier, F-28100 Dreux (FR)
(74) Mandataire: Gallochat, Alain
(86) Numéro de dépôt international: PCT/FR2011/051929
(87) Numéro de publication internationale: WO 2012/022919

(56) Documents cités:
- WO-A2-03/028703
- US-A- 4 282 215
- US-A- 4 366 159
- US-A1- 2009 030 026
- LO, M. W. ET AL: "The disposition and bioavailability of intravenous and oral nalbuphine in healthy volunteers", JOURNAL OF CLINICAL PHARMACOLOGY, LIPPINCOTT CO, HAGERSTOWN, MD, US, vol. 27, 1 janvier 1987 (1987-01-01), pages 866-873, XP009143126, ISSN: 0091-2700
- AUNGST, B.J. ET AL: "Oral and rectal nalbuphine bioavailability: first-pass metabolism in rats and dogs.", BIOPHARM DRUG DISPOS, vol. 6, 1985, pages 413-421, XP002616787,

## Description

La présente invention a pour objet une nouvelle formulation pharmaceutique contenant de la nalbuphine ainsi que son utilisation dans le traitement de la douleur.

La douleur est une affection très fréquente et correspond à l'un des symptômes le plus couramment traité en thérapeutique.

Des millions d'individus souffrent ainsi continuellement de douleurs récurrentes sans recevoir de traitement totalement satisfaisant en terme d'amélioration des symptômes.

Les dérivés de la morphine ont été largement utilisés dans le traitement de la douleur par le passé et leur usage s'est encore considérablement accru dans les dix dernières années.

La nalbuphine est un dérivé opiacé hémisynthétique de type agoniste des récepteurs κ et antagoniste des récepteurs µ, appartenant à la série des phénanthrènes. Cette molécule est inscrite au palier IIA de l'OMS. Elle présente une activité analgésique équivalente à celle de la morphine et dix fois supérieure à celle de la pentazocine.

Elle est également utilisée en anesthésie. Son autre intérêt réside dans la moindre prévalence de dépression respiratoire et de dépendance comparativement aux autres dérivés opiacés.

Les propriétés suivantes font, en effet, de la nalbuphine un analgésique idéal : rapidité d'action, intensité d'action, peu d'effet inhibiteur sur le système cardiovasculaire et sur le système respiratoire et pas d'effet addictogène.

Toutefois, si la nalbuphine est bien absorbée par voie intraveineuse (IV), l'absorption par voie orale est faible et variable, ceci en raison d'un fort effet de premier passage hépatique. Ainsi, les formes actuellement commercialisées de nalbuphine sont destinées aux voies parentérale, intramusculaire ou rectale avec les inconvénients et effets secondaires que supposent ces voies d'administration.

Différentes compositions pharmaceutiques contenant de la nalbuphine ont été précédemment étudiées et décrites.

La demande internationale WO 82/03768 décrit une forme nasale composée d'une solution, une suspension ou une pommade. Il n'est pas fait mention de forme orale.

Les demandes internationales WO 98/0951 et WO 00/24386 décrivent des patchs transdermiques de principe actif et notamment de chlorhydrate de nalbuphine.

Le brevet US 6680067 concerne une composition pharmaceutique à libération contrôlée, se présentant sous forme d'une suspension huileuse, comprenant un composé analgésique qui est la nalbuphine sous forme de base ou de sel, en mélange avec une huile injectable. Cette composition est caractérisée en ce qu'elle comprend des microparticules présentes dans la suspension huileuse, de taille inférieure à 100 µm, obtenues par un appareil de mélange à très haute énergie, permettant d'obtenir des particules de taille inférieure à 100 microns.

La demande de brevet Russe RU 02/254852 décrit une solution injectable de chlorhydrate de nalbuphine.

La demande internationale WO 2007/025005 concerne des formulations à libération prolongée de nalbuphine ou de l'un de ses sels pharmaceutiquement acceptables, destinées à une administration par voie orale ; cette demande décrit des compositions comprenant de la nalbuphine ou l'un de ses sels et un système de délivrance à libération prolongée, le-dit système comprenant au moins un composé hydrophile, au moins un agent réticulant et au moins un diluant pharmaceutique ou, au moins un composé hydrophile, au moins un agent réticulant, au moins un diluant pharmaceutique et au moins un agent réticulant cationique différent du 1 er agent réticulant ou au moins un composé hydrophile, au moins un agent réticulant cationique et au moins un diluant pharmaceutique.

De même, la demande internationale WO 2005/127683 concerne des formulations, essentiellement à libération prolongée, de nalbuphine ou de l'un de ses sels pharmaceutiquement acceptables, destinées à une administration par voie orale. Additionnel art anterieur comprend US4366159.

Un des objets de l'invention ici présentée est de proposer au clinicien une forme orale de nalbuphine présentant une action rapide proche de la forme IV et suffisamment élevée.

En effet, il n'existe pas, actuellement, de formulation immédiate orale, car il a été observé, que les formulations immédiates testées jusqu'alors, montraient une faible biodisponibilité, ceci en conséquence de l'effet bolus qui provoque une saturation du système enzymatique hépatique (cytochrome p 450), d'où les recherches intensives pour développer plutôt une forme à libération prolongée.

Une autre difficulté pour l'utilisation d'une forme orale de nalbuphine, réside dans le goût particulièrement désagréable et intense du principe actif, ce qui empêche son utilisation directe en solution. Cela suppose alors un masquage de goût par les artifices galéniques habituels (aromatisation et enrobage) qui vont nuire à une libération immédiate du principe actif.

Les recherches ont donc plutôt porté sur des formulations à libération prolongée.

La présente invention a pour but de fournir une nouvelle formulation pharmaceutique orale, solide, comprenant de la nalbuphine permettant une solubilisation rapide et une biodisponibilité équivalente à une forme soluté de nalbuphine.

Ainsi, la présente invention concerne une formulation pharmaceutique orale à libération immédiate comprenant de la nalbuphine ou l'un de ses sels pharmaceutiquement acceptables et au moins un support de granulation hydrophile, un liant hydrophile et un lubrifiant.

On entend par « hydrophile », toute substance soluble ou dispersible dans l'eau ou dans un solvant polaire.

La formulation pharmaceutique, selon l'invention, contient uniquement de la nalbuphine ou l'un de ses sels pharmaceutiquement acceptables en tant que principe actif, de préférence la nalbuphine chlorhydrate.

La présente invention découle de la mise en évidence inattendue par les inventeurs qu'une formulation pharmaceutique orale à libération immédiate, contenant de la nalbuphine et au moins un support de granulation hydrophile, un liant hydrophile et un lubrifiant permet d'obtenir une bonne solubilité et donc une biodisponibilité équivalente à celle d'une forme solution de nalbuphine pour injection administrée per os. La formulation selon l'invention a donc une efficacité dans le traitement de la douleur équivalente à celle d'une formulation pour injection et permet la libération immédiate du composé actif afin de traiter rapidement la douleur.

La présente invention décrit donc une formulation orale, à libération immédiate de nalbuphine permettant d'obtenir des résultats *in vivo* comparables à ceux d'une formulation injectable de nalbuphine en solution administrée oralement.

L'expression formulation pharmaceutique « à libération immédiate » comprend toutes les formulations pharmaceutiques dont le taux de libération et d'absorption n'est pas modifié, en particulier sans effet de libération prolongée par une quelconque manipulation galénique. Dans le cas présent, la libération immédiate est obtenue par l'utilisation d'au moins un support de granulation hydrophile, un liant hydrophile et un lubrifiant appropriés pharmaceutiquement qui ne prolongent pas sensiblement la libération ou l'absorption de la formulation. L'expression « à libération immédiate » exclut les formulations adaptées pour une libération dite « prolongée », « retardée » ou « contrôlée ». Préférentiellement, une forme à libération immédiate est une forme pour laquelle la quantité de substance libérée est d'au moins 75% en quarante cinq minutes (« guidance for industry, dissolution testing of immediate release solid dosage forms 1997, FDA, CDER").

La formulation selon l'invention ne contient pas d'agent réticulant capable, en présence d'un composé hydrophile, de former une matrice utilisable pour une libération retardée du principe actif. Par agent réticulant on entend par exemple les homo-polysaccharides dont les gommes de guar, les gommes de caroube, ou des agents réticulants cationiques.

Une formulation peut être caractérisée par sa cinétique de libération *in vitro.* Préférentiellement, une formulation à libération immédiate selon l'invention présente une cinétique de libération *in vitro* égale ou supérieure à 80% en 45 minutes.

L'expression « nalbuphine ou l'un de ses sels" inclut selon l'invention la nalbuphine, chlorhydrate/HCl de nalbuphine, les esters de nalbuphine ainsi que leurs sels pharmaceutiquement acceptables, leurs complexes et dérivés. Préférentiellement, la nalbuphine selon l'invention est sous forme de chlorhydrate.

Le terme "pharmaceutiquement acceptables" se rapporte à des molécules et formulations qui n'induisent pas une réaction adverse, allergique ou non désirée lorsqu'elles sont administrées à un mammifère, en particulier à un humain.

La formulation selon l'invention comprend un support de granulation hydrophile, un liant hydrophile et un lubrifiant. Préférentiellement, le support de granulation hydrophile, le liant hydrophile et le lubrifiant sont choisis de manière à permettre une solubilisation très rapide du principe actif permettant ainsi de traiter rapidement la douleur. Préférentiellement, le support de granulation hydrophile, le liant hydrophile et le lubrifiant sont choisis de manière à ne pas modifier la libération du principe actif en fonction des variations de pH, ce qui permet une prise des formulations selon l'invention indépendante des prises alimentaires, qui peuvent faire varier le pH gastrique.

En effet, les formulations selon l'invention conduisent à une libération quasi « flash » du principe actif.

L'expression « support de granulation » comprend tous les composés permettant la granulation de la formulation pharmaceutique selon l'invention.

Par « granulation », on entend tous les procédés permettant de transformer des particules de poudre cristallisées ou amorphes en agrégats solides plus ou moins résistants et plus ou moins poreux.

Préférentiellement, le support de granulation hydrophile est choisi dans le groupe comprenant des polyols, par exemple, le mannitol, le sorbitol, le maltitol ou le xylitol, le lactose, le phosphate dicalcique, un carbonate par exemple le carbonate de calcium, de potassium, de magnésium ou de sodium, un gluconate, un dérivé de la silice par exemple un silicate comme le silicate de magnésium aluminium (Neusilin® UFL2), des cristaux de sucre, les dérivés de I amidon , le saccharose, le polyvinyl pyrrolidone (PVP) ou un de ses dérivés, un dérivé de la cellulose, par exemple la méthylcellulose, l'hydroxypropyl méthylcellulose ou la carboxy-méthylcellulose, le polyéthylène glycol ou un de ses dérivés, seuls ou en mélange.

Encore préférentiellement, le support de granulation hydrophile selon l'invention est choisi parmi le mannitol, le polyvinyl pyrrolidone ou un de ses dérivés, un polyol, le lactose, un dérivé de la cellulose par exemple la méthylcellulose, l'hydroxypropyl méthylcellulose ou la carboxyméthylcellulose, le polyéthylène glycol ou un de ses dérivés et un dérivé de la silice.

Dans un mode de réalisation préféré, le support de granulation est le lactose.

L'expression « au moins un support de granulation » indique que la formulation pharmaceutique selon l'invention peut comprendre un, deux, trois ou plus, supports de granulation différents.

La formulation selon l'invention comprend également au moins un liant hydrophile. Le liant selon l'invention peut être choisi parmi une gomme, par exemple une gomme arabique ou adragante, la gélatine, les amidons, les maltodextrines, le polyéthylène glycol par exemple le PEG 4000 ou 6000, le PVP ou un de ses dérivés, les solutions de saccharose, de glucose ou de sorbitol, un polyol, le lactose, les carbomères, un dérivé de la cellulose, par exemple la méthylcellulose, l'hydroxypropyl méthylcellulose ou la carboxyméthylcellulose.

Préférentiellement, le liant est choisi parmi le groupe comprenant le PVP ou un de ses dérivés, un polyol, le lactose, un dérivé de la cellulose, par exemple la méthylcellulose, l'hydroxypropyl méthylcellulose ou la carboxyméthylcellulose, le polyéthylène glycol ou un de ses dérivés, les gommes, par exemple les gommes arabique et adragante, et les carbomères.

Dans un mode de réalisation préféré, le liant est le PVP.

L'expression «au moins un liant hydrophile» indique que la formulation pharmaceutique selon l'invention peut comprendre un, deux, trois ou plus, liants différents.

La formulation comprend également au moins un lubrifiant. Le lubrifiant selon l'invention peut être choisi parmi la silicone dioxyde, le stéarate de magnésium, la silice anhydre colloïdale, le stéarylfumarate de sodium ou le talc.

L'expression «au moins un lubrifiant» indique que la formulation pharmaceutique selon l'invention peut comprendre un, deux, trois ou plus, lubrifiants différents. Le(s) lubrifiant(s) sont choisis de telle sorte qu'ils n'ont pas d'influence sur la cinétique de libération du(des) principe(s) actif(s).

Dans un mode de réalisation préféré, la formulation comprend trois lubrifiants différents.

La formulation selon l'invention peut contenir d'autres composés comme par exemple au moins un excipient. Préférentiellement, l'excipient pouvant être utilisé dans les formulations selon l'invention est choisi parmi les agents d'aromatisation ou de masquage de goût, les édulcorants, les excipients de coloration et les agents désintégrants, tels que la polyvinylpyrrolidone. Les agents d'aromatisation et de masquage de goût ainsi que les édulcorants sont particulièrement souhaitables dans les formulations selon l'invention afin de supprimer ou d'atténuer le goût désagréable de la nalbuphine

On entend par "agent d'aromatisation" tout substance utilisée dans l'industrie pharmaceutique destinée à être introduite dans une formulation de manière à en modifier ou masquer la saveur ou l'odeur. On pourra également être amené à utiliser des édulcorants qui sont des produits naturels ou synthétiques présentant une saveur sucrée. Préférentiellement, on utilisera les édulcorants acceptables pharmaceutiquement comme les succédanés du sucre (dérivés des polyols, dérivés naturels : stevia, etc) et les édulcorants intenses (aspartam, cyclamate, saccharine etc.).

La formulation selon l'invention peut également contenir au moins un agent filmogène afin en particulier de réaliser un enrobage cohésif, pour augmenter la stabilité de la formule ou pour masquer le goût. Préférentiellement l'agent filmogène est choisi parmi les dérivés de la cellulose, l'HPMC, les dérivés du polyéthylène glycol, les dérivés du polyvinyl pyrrolidone, les cires, les dérivés acryliques (par exemple Eudragit® L, RL, S) et les carbomères. L'agent filmogène est plus particulièrement choisi de façon à masquer le goût tout en n'agissant pas sur la cinétique de libération du principe actif. L'agent filmogène est préférentiellement hydrophile.

La formulation selon l'invention représente une combinaison particulière d'excipients qui permettent d'obtenir une forme orale à libération à « effet flash ». Cette libération à « effet flash » permet d'obtenir une libération très rapide du(des) principe(s) actif(s). En effet, avec la formulation selon l'invention, la quantité de substance libérée est d'au moins 95% en quinze minutes. Pour une forme à libération immédiate conventionnelle, la quantité de substance libérée est d'au moins 75% en quarante cinq minutes (« guidance for industry, dissolution testing of immediate release solid dosage forms 1997, FDA, CDER").

Dans un mode de réalisation préféré, la formulation selon l'invention comprend en outre:
- au moins un agent désintégrant ;
- au moins un excipient.

La formulation selon l'invention peut être complétée par des excipients, par exemple, un ou des agent(s) de compression.

Dans certains modes de réalisation préférés, la formulation pharmaceutique selon l'invention comprend moins de 30% en poids de nalbuphine, préférentiellement de 4 à 30% en poids de nalbuphine et toujours préférentiellement de 18 à 22 % en poids de nalbuphine par rapport au poids total de la formulation.

Dans certains modes de réalisation préférés, la formulation pharmaceutique selon l'invention comprend au moins 45% en poids de support de granulation hydrophile, préférentiellement de 45 à 70% en poids et encore préférentiellement de 55 à 65 % en poids de support de granulation hydrophile par rapport au poids total de la formulation.

Dans certains modes de réalisation préférés, la formulation pharmaceutique selon l'invention comprend moins de 10% en poids de liant hydrophile, préférentiellement de 2 à 10% en poids et toujours préférentiellement de 4 à 8 % en poids de liant par rapport au poids total de la formulation.

Dans certains modes de réalisation préférés, la formulation pharmaceutique selon l'invention comprend moins de 10% en poids de lubrifiant, préférentiellement de 0,9 à 7% en poids et toujours préférentiellement de 2 à 3 % en poids de lubrifiant par rapport au poids total de la formulation.

Dans un mode de réalisation préféré de l'invention, la formulation comprend particulièrement:
- de 4 à 30% en poids de nalbuphine,
- de 45 à 70% en poids de lactose ;
- de 2 à 10% en poids de PVP; et
- de 0,9 à 7% en poids d'au moins un lubrifiant, complétée avec des excipients.

La formulation pharmaceutique selon l'invention est destinée à une administration orale.

La formulation pharmaceutique selon l'invention est sous forme solide. Les formes solides selon l'invention comprennent en particulier les comprimés, les granules, les granulés, les micro-granules, les capsules, les gélules, les comprimés sublinguaux, les pastilles, les pilules, les poudres, les cachets, les tablettes et les dragées. Préférentiellement, la forme solide selon l'invention est choisie dans le groupe comprenant les comprimés, les granules, les granulés, les micro-granules, les gélules et les pastilles. Les formes de la formulation selon l'invention destinées à être directement avalées par l'utilisateur, telles que les gélules ou les comprimés, sont particulièrement préférées. La forme de comprimé est particulièrement préférée. En effet, ce mode d'administration permet une ingestion de la formulation suffisamment rapide pour que l'utilisateur ne soit pas dérangé par le goût désagréable de la nalbuphine.

Les techniques utilisées pour l'obtention de la forme solide de la formulation selon l'invention peuvent consister en tous les procédés connus par l'homme du métier pour l'obtention de formulations sous forme solide. En particulier, ces procédés peuvent être choisis parmi les techniques de granulation sèche ou humide, la compression directe, la granulation en lit d'air fluidisé, le pelliculage et le spray drying.

La formulation selon l'invention permet préférentiellement une bonne biodisponibilité de la nalbuphine. Par « bonne biodisponibilité », on entend qu'une proportion suffisante de la nalbuphine contenue dans la formulation va effectivement agir dans l'organisme par rapport à la quantité absorbée et apporter l'effet thérapeutique recherché.

Dans un mode de réalisation particulier, l'invention concerne la formulation pharmaceutique telle que définie ci-dessus pour son utilisation comme médicament dans le traitement de la douleur, préférentiellement dans le traitement des douleurs non chroniques.

L'avantage des formulations à libération immédiate selon l'invention pour une telle utilisation est qu'elles permettent un traitement rapide de la douleur contrairement aux formulations à libération prolongée.

Les douleurs pouvant être traitées par les formulations pharmaceutiques selon l'invention peuvent être dues à n'importe quelle cause par exemple la douleur engendrée par un traitement de la dépendance aux opioïdes, par le cancer, par des maladies auto-immunes, par des infections et par des traumatismes, etc.

Préférentiellement, la douleur traitée par la formulation selon l'invention est engendrée par un traitement de la dépendance aux opioïdes.

Dans un mode de réalisation particulier, l'invention concerne la formulation pharmaceutique telle que définie ci-dessus pour son utilisation comme médicament dans le traitement de la dépendance aux opioïdes.

### DESCRIPTION DES FIGURES

La Figure 1 représente les différents profils de dissolution de la nalbuphine en fonction du pH.
La Figure 2 représente le pourcentage de nalbuphine dissous en fonction du temps pour la formulation de l'exemple 5, selon différentes conditions opératoires.

### EXEMPLES

### Obtention des formulations pharmaceutiques sous forme de comprimés

Les compositions et procédés d'obtention de 9 types de formulations (exemple 1, exemple 2, exemple 3, exemple 4, exemple 5, exemple 6, exemple 7, exemple 8, exemple 9) sont décrits ci-dessous :

### Exemple 1 : comprimés à 30 mg

**Formule**

| **Matières Premières** | ***Unitaire (mg)*** | ***%*** |
|---|---|---|
| Nalbuphine/HCl | 30.00 | 4.69 |
| Povidone (PVP) | 242.01 | 37.80 |
| Mannitol (support de granulation) | 349.49 | 54.58 |
| Crospovidone | 12.81 | 2.00 |
| Silice anhydre colloïdale | 0.64 | 0.10 |
| stéarylfumarate de sodium | 3.20 | 0.50 |
| Talc | 2.14 | 0.33 |
| **TOTAL** | **640.29** | **100.00** |

Cette formulation a été obtenue en suivant le protocole ci-dessous :
- pesée des matières premières (balance) ;
- mélange de la nalbuphine, d'eau purifiée et de povidone (agitateur hélicoïdal) ;
- solubilisation et complexation du mélange (lit d'air fluidisé GPCG3, Würster et pompe péristaltique) ;
- recouvrement du mannitol par le mélange et séchage du mannitol ainsi recouvert pour obtenir des granules ;
- tamisage des granules (Tamis de 600µm de diamètre, tamis oscillant, tamis de 630, 500 et 400 µm) ;
- lubrification des granules par ajout de talc (mixeur Erweka et réservoir cubique de 8 L) ;
- mélange et lubrification des granules avec le mannitol, le povidone, le crospovidone, la silice anhydre colloïdale et le stéarylfumarate de sodium (mixeur Erweka et réservoir cubique de 8 L) ;
- obtention de comprimés à partir du mélange (Presse rotative SVIAC RP2080, poinçons de 13 mm).

### Exemple 2 : comprimés à 30 mg

**Formule**

| **Matières Premières** | ***Unitaire (mg)*** | **%** |
|---|---|---|
| Nalbuphine/HCl | 30.00 | 18.65 |
| Talc | 4.47 | 2.70 |
| Silice anhydre colloïdale | 0.74 | 0.45 |
| Avicel PH 200 (support de granulation) | 23.17 | 14.00 |
| Tablettose 80 (support de granulation) | 79.44 | 48.00 |
| Povidone | 7.45 | 4.50 |
| Stéarate de Magnésium | 0.74 | 0.45 |
| Sepifilm LP014 | 14.89 | 9.00 |
| Crospovidone | 3.72 | 2.25 |
| Alcool 96° Surfin Pharma | / | / |
| Eau purifiée | / | / |
| **TOTAL** | **320.15** | **100.00** |

Cette formulation a été obtenue en suivant le protocole ci-dessous :
- pesée des matières premières (balance) ;
- Mélange de la nalbuphine, du talc et du Tablettose® et de l'Avicel®;
- Obtention d'une solution liante par mélange de povidone et d'eau purifiée;
- Pulvérisation de la solution liante sur le premier mélange pour obtenir un granulé;
- Séchage du granulé obtenu en lit d'air fluidisé;
- Lubrification par ajout du stéarate de magnésium et de la silice anhydre colloidale; XL, homogénéisation et incorporation au granulé;
- Compression directe du mélange;
- Préparation de la suspension de pelliculage : Sepifilm® LP014 et alcool;
- Pelliculage puis séchage.

### Exemple 3 : comprimés à 10 mg

**Formule**

| **Matières Premières** | ***Unitaire (mg)*** | **%** |
|---|---|---|
| Nalbuphine/HCl | 10,00 | 11,86 |
| Avicel® PH200 (cellulose microcristalline) (support de granulation) | 15,54 | 18,43 |
| Talc | 1,54 | 1,83 |
| Tablettose® 80 (α-lactose-monohydrate aggloméré) (support de granulation) | 44,94 | 53,28 |
| Stéarate de Magnésium | 0,39 | 0,46 |
| Aérosil® 200 (silice) | 0,39 | 0,46 |
| PVP K30 | 3,85 | 4,56 |
| Sepifilm LP014 (enrobage) | 7,70 | 9,13 |
| **TOTAL** | **84.70** | **100.00** |

Cette formulation a été obtenue par le protocole suivant :
- pesée des matières premières (balance) ;
- homogénéisation sur le mélangeur cubique (8 L), 5 minutes à 150tr/min (nalbuphine, Avicel® PH 200, talc, Aérosil® 200, Tablettose® 80 et PVP K30 ;
- lubrification du mélange avec le stéarate de magnésium pendant 1 minute à 150 tr/min ;
- compression du mélange sur Korsch (G009, diamètre 9) ;
- préparation de la solution d'enrobage avec le Sepifilm LP014 et de l'eau purifiée sur une plaque magnétique ;
- enrobage aqueux du mélange compressé sur le LAF GPCG 1.

### Exemple 3-1 : comprimés à 50 mg

| **Matières Premières** | ***Unitaire (mg)*** | **%** |
|---|---|---|
| Nalbuphine/HCl | 51,60 | 20,55 |
| Avicel® ph 200 (cellulose microcristalline) (support de granulation) | 119,35 | 47,53 |
| PVP CL | 12,50 | 4,98 |
| lactose | 55,30 | 22,02 |
| Stéarate de Magnésium | 1,25 | 0,50 |
| aromatisant | 10,01 | 3,98 |
| Sepifilm IR 777 | 1,08 | 0,44 |
| **TOTAL** | **251,09** | **100,00** |

Cette formulation a été obtenue par le protocole suivant :
- pesée des matières premières (balance) ;
- homogénéisation sur le mélangeur cubique (8 L), 5 minutes à 150tr/min (nalbuphine, Avicel®, lactose, PVP CL et aromatisant) ;
- lubrification du mélange avec le stéarate de magnésium pendant 1 minute à 150 tr/min ;
- compression du mélange sur Korsch (G009, diamètre 9) ;
- préparation de la solution d'enrobage avec le Sepifilm IR 777, eau purifiée et aromatisant sur une plaque magnétique ;
- enrobage aqueux du mélange compressé sur le LAF GPCG 1.

### Exemple 4 : granulés 10 mg

**Formule**

| **Matières Premières** | ***Unitaire (mg)*** | **%** |
|---|---|---|
| Nalbuphine/HCl | 10.00 | 1.99 |
| Arôme masquant | 146.72 | 29.20 |
| PEG 400 | 139.71 | 27.80 |
| Sucralose (édulcorant) | 18.54 | 3.69 |
| Arôme Caramel | 12.84 | 2.56 |
| Neusilin UFL2 (support de granulation) | 107.00 | 21.29 |
| Povidone K30 (PVP) | 32.61 | 6.49 |
| Sepifilm LP014 | 32.60 | 6.49 |
| Talc | 2.50 | 0.50 |
| **TOTAL** | **502.52** | **100.00** |

Cette formulation a été obtenue par le protocole suivant :
- pesée des matières premières (balance) ;
- dispersion du PEG 400, de la nalbuphine, du sucralose, de l'arôme masquant, de l'arôme caramel dans de l'éthanol (agitateur) ;
- imprégnation (mixeur et pompe péristaltique) du Neusilin UFL2 par ce mélange, calibration (rotor oscillant) et séchage (séchoir à lit fluidifié, réservoir de pulvérisation par le haut) ;
- ajout de PVP 30 et d'eau purifiée et granulation du mélange obtenu (séchoir à lit fluidifié, réservoir de pulvérisation par le haut) ;
- calibration des granulés (rotor oscillant et tamis de 800 µm) ;
- ajout de Sepifilm LP014 et d'eau pour enrober les particules et séchage (granulateur à lit fluidifié, réservoir Würster) ;
- lubrification des particules enrobées avec du talc (mixeur cubique).

### Exemple 5 : comprimés de 30 mg

| Matières Premières | Formule unitaire Quantité en mg | % en poids |
|---|---|---|
| Nalbuphine | 30,00 | 20,31 |
| PVP K30 | 9,83 | 6,65 |
| Polyplasdone XL | 3,52 | 2,38 |
| Avicel PH200 | 1,19 | 1,76 |
| Tablettose® 80 | 91,85 | 62,16 |
| Aérosil® 200 | 0,14 | 0,10 |
| Stéarate de magnésium | 0,71 | 0,48 |
| Talc | 2,83 | 1,92 |
| Sepifilm® LP014 | 7,11 | 4,81 |
| Eau purifiée | / | / |
| Total | 147,75 | 100,00 |

Cette formulation a été obtenue par le protocole suivant :
- Pesée des matières premières (balance);
- Mélange de la nalbuphine, du talc et du Tablettose® (support de granulation);
- Obtention d'une solution liante par mélange de l'eau purifiée avec la PVP K30 ;
- Pulvérisation de la solution liante sur le premier mélange pour obtenir un granulé;
- Séchage du granulé obtenu en lit d'air fluidisé;
- Mélange de l'Avicel®, de l'Aérosil®, et de la Polyplasdone XL, homogénéisation et incorporation au granulé;
- Lubrification du mélange avec ajout du stéarate de magnésium ;
- Compression directe du mélange;
- Préparation de la suspension de pelliculage : Sepifilm® LP014 et eau purifiée;
- Pelliculage puis séchage.

### Exemple 6 : Etude comparative du temps de désintégration des comprimés avec des formulation présentant des supports de granulation différents

**Remplacement du Tablettose 80 par du Glycérophosphate de Calcium**

| | ***Comprimés enrobés*** | | | |
|---|---|---|---|---|
| **Matières Premières** | ***g*** | ***mg*** | ***%*** | ***mg obtenu*** |
| Nalbuphine | 63,978 | 30,865 | 20,659 | 30,947 |
| Talc | 5,880 | 2,837 | 1,899 | 2,844 |
| Glycérophosphate de calcium | 190,522 | 91,913 | 61,520 | 92,157 |
| PVP K30 à 25% | 20,398 | 9,840 | 6,586 | 9,867 |
| Polyplasdone XL | 8,130 | 3,922 | 2,625 | 3,932 |
| Avicel ph 200 | 4,067 | 1,962 | 1,313 | 1,967 |
| Aérosil 200 | 0,332 | 0,160 | 0,107 | 0,160 |
| Stéarate de Mg | 1,634 | 0,788 | 0,528 | 0,790 |
| Sepifilm LP 014 | 14,750 | 7,116 | 4,763 | 7,135 |
| Eau purifiée | 132,72 | - | - | - |
| **TOTAL** | 309,690 | 149,40 | 100,00 | 149,80 |

Les quantités des matières premières sont très proches de celles de la composition de l'exemple 5. Seul le support de granulation a été changé dans cette composition de comprimés enrobés.

Les comprimés selon la composition ci-dessus et des comprimés selon la composition de l'exemple 5 sont testés.

Ils sont soumis à une agitation constante dans un milieu maintenu à 37,0°C (+/-0,5°C) dans un appareil de dissolution. Des prélèvements sont effectués, grâce à une pompe à piston et un collecteur d'échantillons à 5, 10, 15, 30 et 60 minutes. Ces prélèvements sont ensuite analysés par HPLC.

Les conditions de dissolution sont les suivantes :
- Appareil 1 USP <711> (appareil à paniers)
- Vitesse de rotation : 50 tpm
- Milieu de dissolution : HCl 0,1 N
- Volume : 500 mL
- Prise d'essai : un comprimé de 30 mg.

Le temps de désintégration pour ces comprimés enrobés est supérieur à 8h30.

Le temps de désintégration dans les mêmes conditions opératoires pour l'exemple 5 se situe entre 5 et 8 minutes.

### Exemple 7 : Etude de dissolution dans des milieux à pH différents

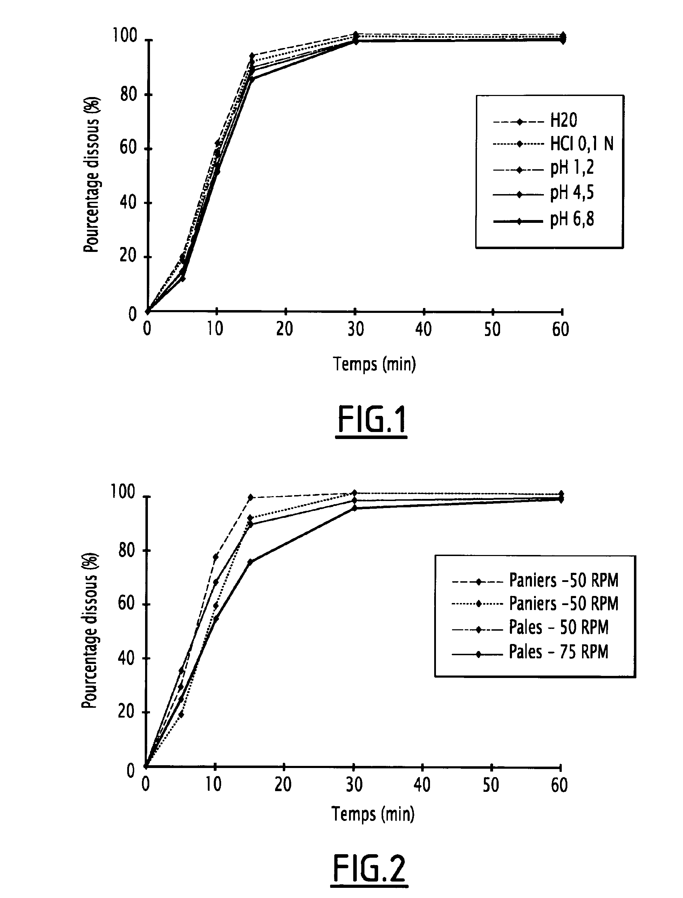

Les conditions opératoires sont identiques à celles utilisées pour l'étude comparative précédente.
Au cours de cette étude, plusieurs milieux à différents pH sont testés : HCl 0,1 N, à pH 1,2 ; 4,5 et 6,8 (Figure 1).
On constate que le pH et la nature du milieu n'ont pratiquement pas d'influence sur le profil de dissolution de la formulation.

### Exemple 8 : Solubilité des formulations

L'augmentation de la biodisponibilité des formulations est estimée en fonction de la dissolution de la forme pharmaceutique.
Une forme à libération immédiate est une forme pour laquelle la quantité de substance libérée est d'au moins 75% en quarante cinq minutes (« guidance for industry, dissolution testing of immediate release solid dosage forms 1997, FDA,CDER"). Les profils de dissolution des différentes formulations sont obtenus par des mesures de dissolution.
Pour cela, un appareil à dissolution équipé de pales est utilisé. Les formulations sont soumises à une agitation constante de 50 tpm, dans un vase contenant 500 ml d'eau purifiée et maintenu à 37°C.
Les prélèvements sont réalisés à des temps prédéterminés, pendant 1 heure puis analysés par HPLC avec détection dans l'ultraviolet, à la longueur d'onde de 285 nm.

### Formulation de l'exemple 1

| | Temps (min) | 5 | 15 | 30 | 45 |
|---|---|---|---|---|---|
| % dissous | Nalbuphine | 84 | 96 | 97 | 97 |

### Formulation de l'exemple 3

| | Temps (min) | 5 | 15 | 30 |
|---|---|---|---|---|
| % dissous | Nalbuphine | 84 | 93 | 96 |

### Formulation de l'exemple 4

| | Temps (min) | 5 | 10 | 15 |
|---|---|---|---|---|
| % dissous | Nalbuphine | 97 | 99 | 98 |

Comme le montre les résultats de ces expériences, les différentes formulations présentées ont été spécifiquement développées pour obtenir des dissolutions très rapides, conformes à la définition.

### Exemple 9 : Etude pharmacocinétique

Une étude de phase 1, ouverte, à deux bras, a été menée sur les formulations suivantes :
- formulation orale de l'Exemple 1 (comprimés 30 mg) ;
- forme intraveineuse (IV) de nalbuphine administrée oralement.

Une étude a été réalisée comprenant l'administration d'une dose unique à 8 volontaires sains masculins à jeun (4 volontaires par bras). Différents paramètres ont été mesurés : Tmax (temps au bout duquel la quantité maximale de formulation est dissoute), Cmax (quantité de formulation maximale dissoute) et AUC (surface située sous la courbe concentration/temps). Les résultats suivant ont été obtenus :

**Tableau 1: Tmax basé sur les valeurs moyennes**

| Formulation | Tmax (h) |
|---|---|
| Forme orale solide de l'exemple 1, 30 mg | 1.25 |
| Solution orale 30 mg (pour comparaison) forme IV administrée per os | 0.75 |

On peut constater que le Tmax est légèrement décalé pour la forme orale solide selon l'invention comparativement à la solution liquide de nalbuphine.

**Tableau 2 : Paramètres pharmacocinétiques, valeur moyenne arithmétique**

| Formulation | Cmax (ng/mL) | Tmax (h) | AUCt (ng/mL*h) | AUCinf (ng/mL*h) |
|---|---|---|---|---|
| Forme orale solide de l'exemple 1, 30 mg | 7.91 | 1.21 | 30.93 | 38.08 |
| Solution orale 30mg (pour comparaison) forme IV administrée per os | 9.34 | 0.71 | 33.06 | 39.07 |

On peut constater que hormis le léger décalage pour le Tmax, les paramètres pharmacocinétiques sont comparables pour la forme solide selon l'invention et pour la solution orale.

**Tableau 3: Biodisponibilité relative (basée sur une valeur moyenne arithmétique normalisée à la dose)**

| Formulation | Cmax ratio | AUCt ratio | AUCinf ratio |
|---|---|---|---|
| Forme orale solide de l'exemple 1, 30mg / Solution orale 30 mg (pour comparaison) | 0.85 | 0.94 | 0.97 |

**Tableau 4: Paramètres pharmacocinétiques, valeur moyenne géométrique**

| Formulation | Cmax (ng/mL) | AUCt (ng/mL*h) | AUCinf (ng/mL*h) |
|---|---|---|---|
| Forme orale solide de l'exemple 1, 30 mg | 6.72 | 26.22 | 31.80 |
| Solution orale 30 mg (pour comparaison) | 8.63 | 29.72 | 34.47 |

**Tableau 5: Biodisponibilité relative (basée sur une valeur moyenne géométrique normalisée à la dose)**

| Formulation | Cmax ratio | AUCt ratio | AUCinf ratio |
|---|---|---|---|
| Forme orale solide de l'exemple 1, 30 mg / Solution orale 30 mg (pour comparaison) | 0.78 | 0.88 | 0.92 |

Ces données montrent que la forme orale solide testée présente une biodisponibilité proche de celle observée pour la forme IV administrée oralement ; on observe un léger décalage de Tmax , mais la libération obtenue reste très rapide et cela n'est pas préjudiciable à l'effet thérapeutique recherché.

### Exemple 10 : Etude comparative versus solution orale de référence

Les données de l'étude précédente ont été comparées aux données issues de la bibliographie. En se basant sur la linéarité de la dose administrée, on obtient les valeurs suivantes :

**Tableau 6 : Paramètres pharmacocinétiques (moyennes arithmétique (dose normalisée à 30 mg)**

| Formulation | Cmax (ng/mL) | AUCt (ng/mL*h) | AUCinf (ng/mL*h) |
|---|---|---|---|
| Forme orale solide de l'exemple 1, 30 mg | 7,91 | 30,93 | 38,08 |
| Solution orale de nalbuphine 30 mg | 6,88 | 30,93 | 34,25 |

**Tableau 7: Biodisponibilité relative (basée sur une valeur moyenne arithmétique normalisée à la dose 30 MG)**

| Formulation | Cmax ratio | AUCt ratio | AUCinf ratio |
|---|---|---|---|
| Forme orale solide de l'exemple 1, 30 mg / Solution orale 30 mg (pour comparaison) | 1,15 | 1,00 | 1,11 |

**Tableau 8 : Paramètre pharmacocinétique (valeurs moyennes géométriques normalisées à la dose 30mg)**

| Formulation | Cmax (ng/mL) | AUCt (ng/mL*h) | AUCinf (ng/mL*h) |
|---|---|---|---|
| Forme orale solide de l'exemple 1, 30 mg | 6,72 | 26,22 | 31,80 |
| Solution orale de nalbuphine 30mg | 6,24 | 28,15 | 31,57 |

**Tableau 9: Biodisponibilité relative (basée sur une valeur moyenne geometrique normalisée à la dose 30 MG)**

| | | | |
|---|---|---|---|
| Forme orale solide de l'exemple 1, 30 mg / Solution orale 30 mg (pour comparaison) | 1,08 | 0,93 | 1,01 |

En raisonnant sur les valeurs géométriques moyennes, la biodisponibilité annoncée pour la solution orale de nalbuphine est comparable à celle observée pour la forme solide.

### Exemple 11 : Etude comparative versus solution orale de référence

Une seconde étude de phase 1, à un seul bras, a été menée sur la formulation orale de l'Exemple 2 (comprimés 30 mg)

Cette étude a été réalisée en administrant une dose unique de 30 mg de nalbuphine à 12 volontaires sains masculins à jeun. Différents paramètres ont été mesurés : Tmax (temps au bout duquel la quantité maximale de formulation est dissoute), Cmax (quantité de formulation maximale dissoute) et AUC (surface située sous la courbe concentration/temps).
Les données observées ont été comparées avec les données obtenues lors de l'étude de l'exemple 9 pour la forme intraveineuse (IV) de nalbuphine administrée oralement

| Formulation | Cmax (ng/mL) | Tmax (ng/mL) | AUCinf (ng/mL*h) |
|---|---|---|---|
| Forme orale solide de l'exemple 2, 30 mg | 12.2 | 2.63 | 38.1 |
| Solution orale liquide 30 mg (pour comparaison) | 9.3 | 3.11 | 39.1 |

Ici aussi, les valeurs observées sont semblables. On remarque que les valeurs de Cmax et de l'AUC sont proches. Le Tmax est plus court pour la formulation selon l'exemple 2.

## Revendications

1. Formulation pharmaceutique orale, sous forme de comprimés à 30 mg, à libération immédiate, comprenant, en poids :
- 4,69% de nalbuphine/HCl,
- 54,58% de mannitol,
- 37,8% de povidone,
- 2% de crospovidone,
- 0,10% de silice anhydre colloïdale,
- 0,50% de stéarylfumarate de sodium,
- 0,33% de talc.

2. Formulation pharmaceutique orale, sous forme de comprimés à 30 mg, à libération immédiate, comprenant, en poids :
- 18,65% de nalbuphine/HCl,
- 48% de α-lactose-monohydrate aggloméré (Tablettose ® 80),
- 14% de cellulose microcristalline (Avicel ® PH200),
- 4,50% de povidone,
- 2,25% de crospovidone,
- 0,45% de silice anhydre colloïdale,
- 0,45% de stéarate de magnésium,
- 2,70% de talc,
- 9% de Sepifilm ® LP014.

3. Formulation pharmaceutique orale, sous forme de comprimés à 10 mg, à libération immédiate, comprenant, en poids :
- 11,86% de nalbuphine/HCl,
- 53,28% de α-lactose-monohydrate aggloméré (Tablettose ® 80),
- 18,43% de cellulose microcristalline (Avicel ® PH200),
- 4,56% de povidone (PVP K30),
- 0,46% de silice (Aérosil ® 200),
- 0,46% de stéarate de magnésium,
- 1,83% de talc,
- 9,13% de Sepifilm ® LP014.

4. Formulation pharmaceutique orale, sous forme de comprimés à 50 mg, à libération immédiate, comprenant, en poids :
- 20,55% de nalbuphine/HCl,
- 22,02% de lactose,
- 47,53% de cellulose microcristalline (Avicel ® PH200),
- 4,98% de povidone (PVP CL),
- 0,50% de stéarate de magnésium,
- 3,98% d'aromatisant,
- 0,44% de Sepifilm ® IR 777.

5. Formulation pharmaceutique orale, sous forme de granulés à 10 mg, à libération immédiate, comprenant, en poids :
- 1,99% de nalbuphine/HCl,
- 27,80% de polyéthylèneglycol (PEG 400),
- 21,29% d'aluminosilicate de magnésium (Neusilin ® UFL2),
- 6,49% de povidone K30,
- 0,50% de talc,
- 3,69% de sucralose (édulcorant),
- 29,20% d'arôme masquant,
- 2,56% d'arôme caramel,
- 6,49% de Sepifilm ® LP014.

6. Formulation pharmaceutique orale, sous forme de comprimés à 30 mg, à libération immédiate, comprenant, en poids :
- 20,31% de nalbuphine/HCl,
- 62,16% de α-lactose-monohydrate aggloméré (Tablettose ® 80),
- 6,65% de povidone (PVP K30),
- 2,38% de crospovidone (Polyplasdone XL),
- 1,76% de cellulose microcristalline (Avicel ® PH200),
- 0,10% de silice (Aérosil ® 200),
- 0,48% de stéarate de magnésium,
- 1,92% de talc,
- 4,81% de Sepifilm ® LP014.

7. Formulation pharmaceutique orale, à libération immédiate, **caractérisée en ce qu'**elle correspond à l'une quelconque des revendications 1 à 6, pour son utilisation dans le traitement de la douleur engendrée par un traitement de la dépendance aux opioïdes.

## Patentansprüche

1. Orale pharmazeutische Rezeptur in Form von 30 mg Tabletten mit unmittelbarer Freisetzung, die gewichtsmäßig umfasst:
- 4,69 % Nalbuphin/HCl,
- 54,58 % Mannitol,
- 37,8 % Povidon,
- 2 % Crospovidon,
- 0,10 % kolloidales wasserfreies Siliziumoxid,
- 0,50 % Natriumstearylfumarat,
- 0,33 % Talcum.

2. Orale pharmazeutische Rezeptur in Form von 30 mg Tabletten mit unmittelbarer Freisetzung, die gewichtsmäßig umfasst:
- 18,65 % Nalbuphin/HCl,
- 48 % agglomeriertes α-Lactose-Monohydrat (Tablettose ® 80),
- 14 % mikrokristalline Zellulose (Avicel ® PH200),
- 4,50 % Povidon,
- 2,25 % Crospovidon,
- 0,45 % kolloidales wasserfreies Siliziumoxid,
- 0,45 % Magnesiumstearat,
- 2,70% Talcum,
- 9 % Sepifilm ® LP014.

3. Orale pharmazeutische Rezeptur in Form von 10 mg Tabletten mit unmittelbarer Freisetzung, die gewichtsmäßig umfasst:
- 11,86 % Nalbuphin/HCl,
- 53,28 % agglomeriertes α-Lactose-Monohydrat (Tablettose ® 80),
- 18,43% mikrokristalline Zellulose (Avicel ® PH200),
- 4,56 % Povidon (PVP K30),
- 0,46 % Siliziumoxid (Aérosil ® 200),
- 0,46 % Magnesiumstearat,
- 1,83 % Talcum,
- 9,13 % Sepifilm ® LP014.

4. Orale pharmazeutische Rezeptur in Form von 50 mg Tabletten mit unmittelbarer Freisetzung, die gewichtsmäßig umfasst:
- 20,55 % Nalbuphin/HCl,
- 22,02 % Lactose,
- 47,53 % mikrokristalline Zellulose (Avicel ® PH200),
- 4,98 % Povidon (PVP CL),
- 0,50 % Magnesiumstearat,
- 3,98 % Aromastoff,
- 0,44 % Sepifilm ® IR 777.

5. Orale pharmazeutische Rezeptur in Form von 10 mg Granulat mit unmittelbarer Freisetzung, die gewichtsmäßig umfasst:
- 1,99 % Nalbuphin/HCl,
- 27,80 % Polyethylenglycol (PEG 400),
- 21,29 % Magnesiumaluminiumsilikat (Neusilin ® UFL2),
- 6,49 % Povidon K30,
- 0,50 % Talcum,
- 3,69 % Sucralose (Süßstoff),
- 29,20 % maskierendes Aroma,
- 2,56 % Karamellaroma,
- 6,49 % Sepifilm ® LP014.

6. Orale pharmazeutische Rezeptur in Form von 30 mg Tabletten mit unmittelbarer Freisetzung, die gewichtsmäßig umfasst:
- 20,31 % Nalbuphin/HCl,
- 62,16 % agglomeriertes α-Lactose-Monohydrat (Tablettose ® 80),
- 6,65 % Povidon (PVP K30),
- 2,38 % Crospovidon (Polyplasdone XL),
- 1,76 % mikrokristalline Zellulose (Avicel ® PH200),
- 0,10 % Siliziumoxid (Aérosil ® 200),
- 0,48 % Magnesiumstearat,
- 1,92 % Talcum,
- 4,81 % Sepifilm ® LP014.

7. Orale pharmazeutische Rezeptur mit unmittelbarer Freisetzung, **dadurch gekennzeichnet, dass** sie einem der Ansprüche 1 bis 6 entspricht, für ihre Verwendung bei der Behandlung von Schmerzen, die auf eine Behandlung der Abhängigkeit von Opiaten zurückzuführen sind.

## Claims

1. An immediate-release oral pharmaceutical formulation in 30 mg tablet form, comprising by weight:
- 4.69 % nalbuphine/HCl,
- 54.58 % mannitol,
- 37.8 % povidone,
- 2 % crospovidone,
- 0.10 % colloidal anhydrous silica,
- 0.50 % sodium stearoyl fumarate,
- 0.33 % talc.

2. An immediate-release oral pharmaceutical formulation in 30 mg tablet form, comprising by weight:
- 18.65 % nalbuphine/HCl,
- 48 % agglomerated α-lactose-monohydrate (Tablettose ® 80),
- 14 % microcrystalline cellulose (Avicel ® PH200),
- 4.50 % povidone,
- 2.25 % crospovidone,
- 0.45 % colloidal anhydrous silica,
- 0.45 % magnesium stearate,
- 2.70 % talc,
- 9 % Sepifilm ® LP014.

3. An immediate-release oral pharmaceutical formulation in 10 mg tablet form, comprising by weight:
- 11.86 % nalbuphine/HCl,
- 53.28 % agglomerated α-lactose-monohydrate (Tablettose ® 80),
- 18.43 % microcrystalline cellulose (Avicel ® PH200),
- 4.56 % povidone (PVP K30),
- 0.46 % silica (Aerosil ® 200),
- 0.46 % magnesium stearate,
- 1.83 % talc,
- 9.13% Sepifilm ® LP014.

4. An immediate-release oral pharmaceutical formulation in 50 mg tablet form, comprising by weight:
- 20.55 % nalbuphine/HCl,
- 22.02 % lactose,
- 47.53 % microcrystalline cellulose (Avicel ® PH200),
- 4.98 % povidone (PVP CL),
- 0.50 % magnesium stearate,
- 3.98% flavouring
- 0.44% Sepifilm ® IR 777.

5. An immediate-release oral pharmaceutical formulation in 10 mg granule form, comprising by weight:
- 1.99 % nalbuphine/HCl,
- 27.80 % polyethylene glycol (PEG 400),
- 21.29 % magnesium aluminosilicate (Neusilin ® UFL2),
- 6.49 % povidone K30,
- 0.50 % talc,
- 3.69 % sucralose (sweetener),
- 29.20 % masking flavouring,
- 2.56 % caramel flavouring,
- 6.49 % Sepifilm ® LP014.

6. An immediate-release oral pharmaceutical formulation in 30 mg tablet form, comprising by weight:
- 20.31 % nalbuphine/HCl,
- 62.16 % agglomerated α-lactose-monohydrate (Tablettose ® 80),
- 6.65 % povidone (PVP K30),
- 2.38 % crospovidone (Polyplasdone XL),
- 1.76 % microcrystalline cellulose (Avicel ® PH200),
- 0.10 % silica (Aerosil ® 200),
- 0.48 % magnesium stearate,
- 1.92 % talc,
- 4.81 % Sepifilm ® LP014.

7. An immediate-release oral pharmaceutical formulation **characterized in that** it corresponds to any of claims 1 to 6 for use thereof to treat pain generated by treatment for opioid addiction.
